# EUROPÄISCHE PATENTSCHRIFT

(11) **EP 1 250 911 B1**
(45) Veröffentlichungstag und Bekanntmachung des Hinweises auf die Patenterteilung: **22.09.2004**
(21) Anmeldenummer: 02008218.6
(22) Anmeldetag: 18.04.2002
(51) Int. Cl.: A61K 7/13

(54) **Haarfärbemittel**
Hair dye composition
Teinture pour cheveux

(30) Priorität: 18.04.2001 DE 10118891
(43) Veröffentlichungstag der Anmeldung: 23.10.2002
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- EP-A- 0 685 226
- EP-A- 1 092 415
- WO-A-96/09807
- DE-A- 19 808 066

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobehzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht bzw. noch intensiviert werden können. Auch Tetraaminopyrimidine, insbesondere 2,4,5,6-Tetraaminopyrimidin und Triaminohydroxypyrimidine sowie Dihydroxydiaminopyrimidine (vgl. EP 0 467 026 A1) sind bereits als Entwicktersubstanzen für Haarfärbemittel vorgeschlagen und eingesetzt worden.

WO 96/09 807 A1 offenbart 2-hydroxy-1-ethanon Derivate, beispienveise Acetoin, Hydroxyaceton and Dihydroxyaceton enthaltende Haarfärbezusammensetzungen. Das Dokument beschreibt Verwendung von 2-Hydroxy-1-ethanon Derivate in Abwesenheit von Kuppler- und oxidierenden Substanzen.

Selbst dadurch bleiben jedoch noch färbtechnische Wünsche offen.

Die Erfindung geht daher von der Aufgabenstellung aus, ein Haarfärbemittel zu schaffen, das zur Herstellung einer großen Anzahl von Farbtönen geeignet ist und vor allem eine besonders intensive glänzende Färbung bewirkt.

Diese Aufgabe wird dadurch gelöst, daß ein solches Haarfärbemittel mindestens ein mit Peroxid reagierendes Oxidationsfarbstoffvorprodukt enthält, das ausgewählt ist aus Triaminohydroxypyrimidinen, Diaminomono- und -dihydroirypyrimidinen und/oder Aminotriazinen bzw. deren wasserlöslichen Salzen und mindestens eine Kuppler substanz, in wäßriger Grundlage vorliegt, und 0,01 bis 10 Gew.-% Dihydroxyaceton, berechnet auf die Gesamtzusammensetzung des Mittels, enthält.

Geeignete. Triaminohydroxypyrimidine sind z.B. 4-Hydroxy-2,5,6-tetraaminopyrimidin, 2-Hydroxy-4,5,6-triaminopyrimidin und 5-Hydroxy-2,4,6-triaminopyrimidin; geeignete Mono- und Diaminodihaydroxypyrimidine sind beispielsweise 2,6-Dihydroxy-4,5-diaminopyrimidin, 2,4-Diamino-6-hydroxypyrimidin oder 4,6-Dihydroxy-2,5-diaminopyrimidin bzw. deren wasserlösliche Salze; ein bevorzugtes aminosubstituiertes Triazin ist das 2,4-Diamino-1,3,5-triazin. Das erfindungsgemäße Mittel enthält mindestens eine Kupplersubstanz, die ausgewählt sein kann aus esorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methyldioxybenzol, 2,4-Diamino-3-chlorphenol, 5-Amino-2-methoxyphenol und/oder 1-Methoxy-2-amino-4-(2'-hydroxyethylamino)benzol bzw. deren wasserlöslichen Salzen.
Damit soll jedoch der Zusatz weiterer Entwickler- und Kupplersubstanzen keineswegs ausgeschlossen sein.
Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender weiterer Entwickler- und Kupplersubstanzen noch zu anderen Farbnuancen variiert werden können.
Auch die zusätzliche Mitverwendung weiterer, an sich bekannter Entwicklersubstanzen ist möglich. Es sind hierbei insbesondere noch substituierte p-Phenylendiamine wie 2,5-Diaminotoluol, 2-n-Propyl- bzw. 2-Ethyl-pphenylendiamin, 2,6-Dimethyl-p-phenylendiamin, 2-(2,5-Diaminophenyl)ethanol, 1-Amino-4-bis-(2'-hydroxyethyl)aminobenzol, 2-(2-Hydroxyethylamino)-5-amino-toluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1-β-Hydroxyethyl-2,5-diamino-4-chlorbenzol, 1-β-Hydroxyethyl-2,5-diamino-4-methylbenzol, 2-Methoxy-p-phenylendiamin, N,N-Diethyl-p-phenylendiamin, 1-Amino-4-β-methoxyethylaminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol und/oder 1-Amino-4-β-hydroxypropylaminobenzol, Pyrazol und dessen Derivate wie 1-Hydroxyethyl-4,5-diaminopyrazol, 3,4-Diamino-5-hydroxypyrazol, 3,5-Diaminopyrazol, 3,5-Diamino-pyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol, 3,5-Diamino-1,2,4,-triazol, 2-Aminophenol, 4-Aminophenol und dessen Derivate wie 4-Amino-3-methylphenol, 2-Chlor-4-aminophenol, 2,6-Dichlor-4-aminophenol, 2,4-Diaminophenol, 2,6-Dibrom-4-aminophenol, 5-Aminosalicylsäure und/oder 1,2,4-Triaminobenzol bzw. deren wasserlösliche Salze zu erwähnen.
Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.
Das bevorzugte Gewichtsverhältnis der genannten Entwicklersubstanzen zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1.
Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,01 bis 5,0 %, vorzugsweise 0,05 bis 4 %, insbesondere 0,1 bis 3 Gew.-% der Gesamtzusammensetzung (ohne Oxidationsmittel), wobei sich die Angaben jeweils auf den Anteil an freier Base beziehen. Der Anteil an Dihydroxyaceton liegt vorzugsweise bei etwa 0,05 bis 5, vor allem 0,25 bis 2,5, insbesondere bei etwa 0,5 bis 2 Gew.-% des Färbemittels (ohne Oxidationsmittelzusammensetzung).
Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.
Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 12-prozentiger Konzentration.
Es können jedoch auch andere Peroxide wie Harnstoffperoxid und Melaminperoxid eingesetzt werden.
Alternativ zur Peroxidoxidation kann auch eine Luftoxidation vorgenommen werden, beispielsweise, indem eine ein Oxidationsfarbstoffvorprodukt enthaltende Zusammensetzung als Aerosolschaum auf das Haar aufgebracht und dort für etwa 15 bis 30 Minuten einwirken gelassen wird.
Der pH-Wert des applikationsfertigen Haarfärbemittels, d. h. nach Vermischung mit Peroxid, kann sowohl im schwach sauren, z.B.. einem Bereich von 5,5 bis 6,9, im neutralen als auch im alkalischen Bereich, d. h. zwischen pH 7,1 und 11 liegen.

Im folgenden werden verschiedene Ausführungsbeispiele zur Erläuterung der Erfindung gegeben.

### Grundlage

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | ad 100,00 |

Die erfindungsgemäßen Oxidationsfarbstoff-Kombinationen und Dihydroxyaceton wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

| | 1 | 2 | 3 | 4 | 5 |
|---|---|---|---|---|---|
| 4-Hydroxy-2,5,6-triaminopyrimidinsulfat | 0,61 | 0,61 | 0,61 | | |
| 2,4-Diamino-6-hydroxypyrimidin | | | | 0,28 | 0,28 |
| m-Aminophenol | | 0,25 | | | |
| 2-Methylresorcin | | | 0,28 | | |
| 4-Amino-2-hydroxytoluol | | | | | 0,27 |
| 2-Amino-4-hydroxyethylaminoanisolsulfat | | | | 0,60 | |
| 1-Naphthol | 0,32 | | | | |

| **Färbeergebnisse:** | **Ohne Dihydroxyaceton** | **Mit 0,5% Dihydroxyaceton** |
|---|---|---|
| 1 | Blaßvioiett | Intensives Grauviolett |
| 2 | Rotlila | Intensives Graumagenta |
| 3 | Pastellrosa | Intensives Graurosa |
| 4 | Beige | Braunbeige |
| 5 | Gelbweiß | Orange |

In die beschriebene Grundlage wurden die folgenden Oxidationsfarbstoffmischungen jeweils mit und ohne 0,5 Gew.-% Dihydroxyaceton eingebracht und der pH-Wert so eingestellt, daß beim Vermischen.mit 2%-iger wäßriger H₂O₂-Lösung im Gewichtsverhältnis 1:1 ein pH-Wert der applikationsfertigen Mischung von 6,8 erreicht wurde.
Die Mischungen wurden wiederum jeweils auf Woll-Läppchen und Strähnen aus gebleichtem Menschenhaar aufgebracht, nach 15-minütiger Einwirkung ausgewaschen und getrocknet und die Färbung bewertet.
Es wurde folgendes Ergebnis erzielt:

| Nr. | 2 | 2a |
|---|---|---|
| | ― | -- |
| 2,4,-Diamino-1,3,5-triazin | 0,25 | 0,25 |
| 4-Amino-2-hydroxytoluol | 0,60 | 0,60 |
| Dihydroxyaceton | 0,50 | ― |
| Färbung | Blau | |

Die erfindungsgemäße Zusammensetzung erzielte eine tiefblaue glänzende, intensive Ausfärbung, die der mattblauen der Zusammensetzung 2a deutlich überlegen waren.

## Patentansprüche

1. Mittel zum Färben von menschlichen Haaren, enthaltend mindestens ein Oxidationsfarbstoffvorprodukt, ausgewählt aus Triaminohydroxypyrimidinen, Diaminomono- und-dihydroxypyrimidinen und/oder Aminotriazinen und deren wasserlöslichen Salzen und mindestens einer Kupplersubstanz, in wäßriger Grundlage, **gekennzeichnet durch** einen Gehalt an 0,01 bis 10 Gew.-% Dihydroxyaceton, berechnet auf die Gesamtzusammensetzung des Mittels.

2. Mittel nach Anspruch 1, enthaltend 4-Hydroxy-2,5,6-triaminopyrimidinsulfat und/oder dessen wasserlösliche Salze.

3. Mittel nach Anspruch 1, enthaltend 2,4-Diamino-6-hydroxypyrimidin und/oder dessen wasserlösliche Salze.

4. Mittel nach Anspruch 1, enthaltend 2,4-Diamino-1,3,5-triazin.

5. Mittel nach Anspruch 1, enthaltend mindestens eine Kupplersubstanz, ausgewählt aus Resorcin, 2-Methylresorcin, 4-Chlorresorcin, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy 3,5-dimethoxypyridin, 5-Amino-3-methylphenol, 6-Amino-3-methylphenol, 3-Amino-2-methylamino-6-methoxypyridin, 2-Amino-3-hydroxypyridin, 5-Amino-2-methoxyphenol, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'hydroxyethyl)amino]-benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dyhydroxynaphthalin, 2,7-Dihydroxynaphthalin, 1-Hydroxy-2-methylnaphthalin, 2,4-Diamino-3-chlorphenol und/oder 1-Methoxy-2-amino-4-(2' hydroxyethylamino)benzol bzw. deren wasserlöslichen Salzen.

6. Mittel nach einem oder mehreren der Ansprüche 1 bis 5, **gekennzeichnet durch** einen Gehalt an 0,05 bis 5 Gew.-% Dihydroxyaceton, berechnet auf die Gesamtzusammensetzung.

7. Verwendung von Dihydroxyaceton in Haarfärbemitteln auf Basis mindestens eines Oxidationsfarbstoffvorprodukts, ausgewählt aus Triaminohydroxypyrimidinen, Diaminomono- und -dihydroxypyrimidinen und/oder Aminotriazinen bzw. deren wasserlöslichen Salzen und mindestens einer Kupplersubstanz.

## Claims

1. Composition for the dyeing of human hair, comprising at least one oxidation dyestuff precursor, selected from triaminohydroxypyrimidines, diamino mono- and dihydroxypyrimidines and/or aminotriazines and the water-soluble salts thereof and at least one coupling substance in aqueous basis, **characterized by** a content of 0.01 % to 10 % by weight of dihydroxy acetone, calculated to the total composition.

2. Composition according to claim 1, comprising 4-hydroxy-2,5,6-triaminopyrimidine sulfate and/or the water-soluble salts thereof.

3. Composition according to claim 1, comprising 2,4-diamino-6-hydroxypyrimidine and/or the water-soluble salts thereof.

4. Composition according to claim 1, comprising 2,4-diamino-1,3,5-triazine.

5. Composition according to claim 1, comprising at least one coupling substance, selected from resorcinol, 2-methyl resorcinol, 4-chlororesorcinol, 2-amino-4-chlorophenol, 5-amino-4-methoxy-2-methylphenol, 2-aminophenol, 3-aminophenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 3-amino-2-methylamino-6-methoxypyridine, 2-amino-3-hydroxypyridine, 5-amino-2-methoxyphenol, 2-dimethyl-amino-5-aminopyridine, 2,6-diaminopyridine, 1,3-diamino- benzene, 1-amino-3-(2'-hydroxy-ethylamino)benzene, 1-amino-3-[bis(2'-hydroxy-ethyl) amino]benzene, α-naphthol, 4,6-dichlororesorcinol, 1,3-diaminotoluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 2,4-diamino-3-chlorophenol, and/or 1-methoxy-2-amino-4-(2'-hydroxyethyl amino)benzene or the water soluble salts thereof.

6. Composition according to one or more of claims 1 to 5, **characterized by** a content of 0.05 % to 5 % by weight of dihydroxy acetone, calculated to the total composition.

7. Use of dihydroxy acetone in hair dyeing compositions on the basis of at least one oxidation dyestuff precursor selected from triaminohydroxypyrimidines, diamino mono- and dihydroxypyrimidines and/or amino-triazines or the water-soluble salts thereof

## Revendications

1. Agent de coloration des cheveux humains, qui contient au moins un précurseur de colorant par oxydation sélectionné parmi les triaminohydroxypyrimidines, les diaminomono- et -dihydroxypyrimidines et/ou les aminotriazines et leurs sels solubles dans l'eau, et d'au moins une substance de couplage, à base aqueuse, **caractérisé par** une teneur de 0,01 à 10 % en poids en dihydroxyacétone par rapport à la composition totale de l'agent.

2. Agent selon la revendication 1, qui contient du sulfate de 4-hydroxy-2,5,6-triaminopyrimidine et/ou ses sels solubles dans l'eau.

3. Agent selon la revendication 1, qui contient de la 2,4-diamino-6-hydroxypyrimidine et/ou ses sels solubles dans l'eau.

4. Agent selon la revendication 1, qui contient de la 2,4-diamino-1,3,5-triazine.

5. Agent selon la revendication 1, qui contient au moins une substance de couplage sélectionnée parmi la résorcine, la 2-méthylrésorcine, la 4-chlororésorcine, le 2-amino-4-chlorophénol, le 5-amino-4-méthoxy-2-méthylphénol, le 2-aminophénol, le 3-aminophénol, le 1-méthyl-2-hydroxy-4-aminobenzène, le 3-N,N-diméthylaminophénol, la 2,6-dihydroxy-3,5-diméthoxypyridine, le 5-amino-3-méthylphénol, le 6-amino-3-méthylphénol, la 3-amino-2-méthylamino-6-méthoxypyridine, la 2-amino-3-hydroxypyridine, le 5-amino-2-méthoxyphénol, la 2-diméthylamino-5-aminopyridine, la 2,6-diaminopyridine, le 1,3-diaminobenzène, le 1-amino-3-(2'-hydroxyéthylaminobenzène, le 1-amino-3-[bis(2'-hydroxyéthyl)amino]-benzène, l'α-naphtène, la 4,6-dichlororésorcine, le 1,3-diaminotoluène, la 1-hydroxynaphtaline, le 4-hydroxy-1,2-méthylènedioxybenzène, la 1,5-dihydroxynaphtaline, la 1,6-dihydroxynaphtaline, la 1,7-dihydroxynaphtaline, la 2,7-dihydroxynaphtaline, la 1-hydroxy-2-méthylnaphtaline, le 2,4-diamino-3-chlorophénol et/ou le 1-méthoxy-2-amino-4-(2')hydroxyéthylamino)benzène et leurs sels solubles dans l'eau.

6. Agent selon l'une ou plusieurs des revendications 1 à 5, **caractérisé par** une teneur de 0,05 à 5 % en poids de dihydroxyacétone par rapport à la composition totale.

7. Utilisation de dihydroxyacétone dans des agents de coloration des cheveux à base d'au moins un précurseur de colorant par oxydation sélectionné parmi les triaminohydroxypyrimidines, les diaminomono- et - dihydroxypyridmidines et/ou les aminotriazines et leurs sels solubles dans l'eau, et d'au moins une substance de couplage.
